(19) **Europäisches Patentamt / European Patent Office / Office européen des brevets**

(11) **EP 4 706 520 A1**

(12) **EUROPEAN PATENT APPLICATION**

(43) Date of publication:
**11.03.2026 Bulletin 2026/11**

(21) Application number: **24198722.1**

(22) Date of filing: **05.09.2024**

(51) International Patent Classification (IPC):
**A61B 5/024** (2006.01)  **A61B 5/026** (2006.01)
**A61B 5/1455** (2006.01)

(52) Cooperative Patent Classification (CPC):
**A61B 5/14551; A61B 5/02416; A61B 5/0261**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **DILLEN, Paulus Henricus Antonius**
  **Eindhoven (NL)**
• **VERKRUIJSSE, Willem**
  **Eindhoven (NL)**
• **VAN GASTEL, Mark Josephus Henricus**
  **5656 AG Eindhoven (NL)**

(74) Representative: **Philips Intellectual Property & Standards**
**High Tech Campus 52**
**5656 AG Eindhoven (NL)**

(54) **METHOD, DEVICE AND SYSTEM FOR DETERMINING VITAL SIGN INFORMATION OF A SUBJECT**

(57)    The present invention relates to a method, device and system for determining vital sign information (42) of a subject (200). The method comprises controlling a light source (10) to emit light signals at one or more wavelengths into tissue (220) of the subject (200); obtaining one or more detection signals (40) from a light detector (20) configured to detect light after travelling from the light source (10) through the tissue (220) of the subject (200) to the light detector (20) along a light path (44); determining one or more time of flight, TOF, signals from the one or more obtained detection signals (40); determining, from the one or more TOF signals, one or more light path characteristics of the light path (44) along which the light has traveled; and determining vital sign information (42) of the subject (200) from the one or more determined light path characteristics.

FIG.1

EP 4 706 520 A1

## Description

FIELD OF THE INVENTION

The present invention relates to a method, a device, a system and a computer program for determining vital sign information of a subject, such as patient or other person.

BACKGROUND OF THE INVENTION

Vital signs such as heart rate, pulse rate, arterial blood oxygen saturation, blood perfusion or respiration provide information about the current health state of a subject. Based on vital signs, medical decisions are made. Vital signs are also used to determine a fitness level or to make fitness training more efficient. It is therefore widespread to monitor vital signs, in inpatient and outpatient care settings, at home or in further health, leisure and fitness settings.

One way to measure vital signs is plethysmography (PPG). PPG is a non-invasive, optical measuring technique which is based on the detection of light having been transmitted through soft tissue of the subject or reflected by the skin of the subject. By comparing the portion of light having been transmitted to the portion of light having been absorbed by the soft tissue, a plurality of vital signs can be determined.

The underlying principle of PPG is that that blood absorbs light more than surrounding tissue. Therefore, blood volume changes in the blood vessels may be detected by measuring the light absorption, wherein an increase in blood volume leads to increased (total) light absorption. The PPG signal therefore shows a time dependent behavior, varying with each cardio-vascular pressure wave. Furthermore, the principle of PPG is based on that the quantity of absorbed blood depends on the oxygen content of the blood. Hemoglobin "loaded" with oxygen ("oxygenated hemoglobin") and hemoglobin with less oxygen ("deoxygenated hemoglobin") have different absorption spectra.

A heart rate (or pulse rate) may be calculated by detecting volume changes in blood vessels due to a cardio-vascular pulse wave traveling through the body of the subject with every heartbeat. As the blood volume of the blood vessels varies with every heartbeat, the portion of light that is transmitted through the tissue varies accordingly. Hence, based on the detection of a time-variant change of light transmission, heart rates may be calculated.

A PPG measuring device (e.g., a pulse oximeter) is usually attached to the skin of the subject, e.g. to a fingertip, earlobe or baby foot. Typically, a pulse oximeter comprises at least one light source such as a LED and a light detector, e.g. photodiode, for detecting the emitted light which is transmitted through the tissue of the patient. For detecting the light transmitted through the tissue, the body part of the subject (e.g., fingertip) is usually placed in between the light source and the light detector.

The light used in conventional PPG devices can be single color, e.g., green light. From one color, already a plurality of vital signs can be derived, such as heart rate, calories burned, sleep hypnogram, etc. Using multi-color PPG devices provides the advantage of a more motion robust determination of vital signs. Additionally, multi-color PPG may allow to determine additional vital signs such as oxygen saturation (SpO2) of the blood.

To measure Sp02, pulse oximeters typically periodically switch between emitting light at two different wavelengths, e.g., red and an infrared light, and measure the transmittance of the same area or volume of the tissue of the light at the two different wavelengths. The detected light over time at each wavelength gives PPG waveforms for red and infrared wavelengths respectively.

Conventional PPG measuring devices are directly attached to the skin of the subject. Therefore, they are referred to as 'contact' PPG devices. Although contact PPG is regarded as a basically non-invasive technique, contact PPG measurement is often experienced as being unpleasant and obtrusive, since the pulse oximeter is directly attached to the subject.

More recently, non-contact, remote PPG devices are used too. Remote PPG utilizes light sources or, in general radiation sources, disposed remotely from the subject. Similarly, also a detector, e.g., a camera or a photo detector, can be disposed remotely from the subject of interest. However, remote PPG devices typically achieve a lower signal-to-noise ratio.

One option to determine a distance of the subject is the use of the time of flight (TOF) devices. Conventional TOF devices comprise a light source, e.g., a LED, and a camera. The light source periodically emits light which is partially reflected by the skin of the subject and detected by the camera. By determining the TOF, which corresponds to the time the light needs to "to fly" from the light source to the skin of the subject and back to the camera, the distance of the subject can be determined.

WO 2016/166651 A1 discloses apparatus and methods to monitor vital signs. A multispectral illumination source produces light having a plurality of wavelengths that illuminate a subject. A processing component, such as a time-of-flight camera system, measures the time the light has taken to travel from the multispectral illumination source to the subject and from the subject to the processing component, calculates range information to localize the subject, and calculates vital signs of the subject utilizing photoplethysmographic (PPG) information derived from the light received from the subject and the range information.

SUMMARY OF THE INVENTION

It is an object of the present invention to improve reliability, accuracy and flexibility of vital sign determination.

In a first aspect of the present invention a method for determining vital sign information of a subject is presented, the method comprising:

controlling a light source to emit light signals at one or more wavelengths into tissue of the subject; obtaining one or more detection signals from a light detector configured to detect light after travelling from the light source through the tissue of the subject to the light detector along a light path; determining one or more time of flight, TOF, signals from the one or more obtained detection signals; determining, from the one or more TOF signals, one or more light path characteristics of the light path along which the light has traveled; and determining vital sign information of the subject from the one or more determined light path characteristics.

In a further aspect of the present invention a device for determining vital sign information of a subject is presented, the device comprising

a control unit configured to control a light source to emit light signals at one or more wavelengths into tissue of the subject; a signal input unit configured to obtain one or more detection signals from a light detector configured to detect light after travelling from the light source through the tissue of the subject to the light detector along a light path; and a processing unit configured to determine one or more time of flight, TOF, signals from the one or more obtained detection signals; determine, from the one or more TOF signals, one or more light path characteristics of the light path along which the light has traveled; and determine vital sign information of the subject from the one or more determined light path characteristics.

In another aspect of the present invention a system for determining vital sign information of a subject is presented, the system comprising

a light source configured to emit light signals at one or more wavelengths into tissue of the subject; a light detector configured to detect light after travelling from the light source through the tissue of the subject to the light detector along a light path and generate one or more detection signals based on the detected light; and a device as disclosed herein that is configured to determine vital sign information of the subject from the one or more detection signals.

In yet further aspects of the present invention, there are provided a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed device, system, computer program and medium have similar and/or identical preferred embodiments as the claimed method, in particular as defined in the dependent claims and as disclosed herein.

The present invention is based on the findings that conventional determination of vital signs, e.g. based on a PPG signal, assumes that the light of all wavelengths follows approximately the same light path through soft tissue of a subject. Thus, conventional methods such as PPG do not take into account light path characteristics of the light travelling through the tissue of the subject.

However, the light paths may differ depending on the wavelength of the light. For example, the penetration depth (PD) into the tissue, sometimes also referred to as characteristic penetration depth (CPD) may depend on the wavelength. Furthermore, the light paths may differ due to a change in the physiological state (e.g., circulatory failure, such as failure of the circulation (reduced perfusion in the periphery where typically pulse oximetry is measured, such as fingers, toes, etc.), perfusion, etc.), hemo-dynamic changes (e.g., the finger to which the PPG measuring device is attached is lifted up or down) or situation changes (temperature, sensor pressure, etc.). This leads to inaccuracy and noise in the PPG signals itself and the extracted vital signs (e.g., SpO2) therefrom. Light path variations may be static or dynamic. Relatively small differences in light paths may be calibrated out for a general population of subjects. However, for a single individual, the assumption of similar light paths may be not true.

Additionally, the position of conventional PPG sensor is restricted to few specific body sites like finger, earlobe and baby foot. At these sites, the light path for light of all wavelengths is limited to the thin, soft tissue at these sites (bounded by the skin and, where applicable, bones). Put in other words, the thin layer of soft tissue works as a guidance for the light, resulting in similar light paths for light of different wavelengths. However, it is desirous to measure vital signs more flexible at alternate body sites, where light can travel more freely through the (thicker) tissue. However, conventional PPG sensors are restricted to the specific body sites as at other body sites, the inaccuracy and the noise of conventional PPG sensors is increased. Therefore, vital sign information determined by conventional PGG sensors at other body sites than finger, earlobe and baby foot is less reliable.

The present invention is based on the idea to use TOF information of light having travelled through the tissue to determine vital sign information of the subject. From one or more TOF signals, one or more light path characteristics (LPC) of the light path along which the light has traveled are determined. Based the determined one or more LPC, which may be a (PPG like) signal, vital sign information of the subject may be determined.

In particular, the present invention is based on the findings that the TOF information correlates with the vital signs. For example, as the blood volume in the tissue modulates with the pulse and blood has specific scattering properties, the scattering of light within the tissue and hence the light path characteristics will modulate with the pulse, too. The more the light is scattered, the longer the light path in the tissue will be.

By taking into account light path characteristics of the light, the present invention allows to determine vital sign information more precisely and reliably. Put in other words, the present invention takes into account the dependence of vital sign information on light path characteristics. For example, vital sign information may be precisely determined independently from a tone of the skin or perfusion. Thus, the present invention allows for a more reliable diagnosis and therefore enables a user such as a clinician or a private individual to set up a suitable therapy, training plan, etc.

Furthermore, the present invention allows to determine vital sign information at various body sites more flexible. In particular, the vital sign information may be determined at body sites which are not suitable for conventional devices (e.g., a PPG device) to extract vital sign information. In particular, the present invention may be applied to alternate body sites where light can travel more freely through (thicker) tissue, where light of different wavelengths experiences a different amount of scattering and have different penetration depths and may thus hit upon different body structures such as veins, arteries, bones, tendons. Thus, the present invention allows for a more flexible and more convenient monitoring of the subject's health state. For example, the present invention allows to determine vital sign information such as SpO2 with a wrist worn device or with internet-connected adhesive health monitoring patches that are applied to the chest before hospital discharge and worn for multiple days.

Thus, the present invention allows to take into account scattering of photons due to inter-person differences such as anatomy and physiology in order to increase the accuracy of vital signs. Inter-person variations may be therefore compensated in an algorithm used to determine the vital sign information.

In the context of the present invention as disclosed therein, the term "vital sign information of the subject" refers to information indicating the status of the subject's vital functions and thus on the subject's state of health. The vital sign information can be a time-dependent signal used to monitor the subject over time. For example, the vital sign information is a metric such as pulse rate.

The term "light path characteristics" refers to information which characterizes the light path along which light has travelled through the tissue of the subject. In particular, the light path characteristics characterize the paths of one or more photons of a light signal.

The term "light path" refers to the path along which a photon travels through the tissue of the subject. Additionally, the light path may include the path which the photon takes from the light source up to the skin of the subject and/or the path which the photon takes from the skin of the subject to the light detector.

The term "light signal" refers to one or more photons being emitted. The photons may be emitted, e.g., periodically. The light signal may have a certain "shape" such as a square wave.

The term "one wavelength" refers to light of exact one wavelength (e.g., emitted by a laser) or almost one wavelength (i.e., light with a relatively small spectral range, e.g., such as emitted by a LED). For example, the light signal may comprise red light red only.

The term "(one or) more wavelengths", i.e., a plurality of wavelengths, refers to a light signal comprises more than one (exact) wavelength, wherein a difference between the different wavelengths is not negligible. For example, the light signal comprises green light, red light and infrared light.

The term "time of flight" (TOF) refers to the time a light signal needs to travel from the light source through the tissue of the subject to the light detector. It should be noted that for a plurality of photons, each photon may travel along a different light path. Hence, each photon may have a different TOF. Thus, for each light signal, or for each plurality of photons, a common TOF may be determined. The common TOF may correspond to an average TOF of all photons.

In one embodiment, determining one or more light path characteristics (LPC) includes determining one or more of path length information indicating the path length of the light path along which the light has traveled, path length difference information indicating differences between path lengths of the light paths along which light of different wavelengths has travelled, tissue penetration depth information indicating the penetration depth of light into the tissue, and tissue penetration difference information indicating differences between penetration depths into the tissue of light of different wavelengths.

Therefore, the LPC may be determined based the length of the path light has travelled through the tissue and/or the penetration depth of light into the tissue of the light. Furthermore, the LPC may be determined based on the characteristics of light at one wavelength and/or based on the differences of characteristics of light at different wavelengths (e.g., red light and infrared light).

In an embodiment, determining the one or more light path characteristics comprises taking into account one or more of a peak time at which a maximum of photons of one of the emitted light signals has been detected by the light detector; and a maximum number of photons detected at the peak time.

The peak time is indicative of the TOF of the maximum number of photons. The LPC, e.g., a tissue penetration depth, may be calculated using the product of peak time and the maximum number of photons.

In another embodiment, determining vital sign information includes determining or improving the determination of one or more of pulse rate, oxygen saturation, blood perfusion and variations of blood perfusion.

In a further embodiment, pulse rate, as vital sign information, is determined by detecting periodic variations of a path length from the one or more determined light path characteristics or by detecting variations of a penetration depth of light into the tissue from the one or more determined light path characteristics.

The path length and/or the penetration depth varies with volume changes of blood in the blood vessels. With each cardiovascular wave, the blood volume changes and thus the path length and/or penetration depth changes too. By detecting the path length and/or penetration depth over time, the pulse rate may be monitored over time.

In one embodiment, oxygen saturation, as vital sign information, is determined or its determination is improved by detecting differences between path lengths of the light paths along which light of different wavelengths has travelled or by detecting differences between penetration depths into the tissue of light of different wavelengths.

Preferably, the light source emits red light and infrared light, wherein path lengths of the red light and infrared light each are detected and used to calculate the oxygen saturation.

In another embodiment, variations of blood perfusion, as vital sign information, is determined by detecting the path length of the light paths along which light has travelled or by detecting the penetration depth of light into the tissue. With increasing perfusion, the path length and the tissue penetration depth increases. Conversely, with decreasing perfusion, the path length and the penetration depth decreases, too. Thus, by detecting the path length and/or the penetration depth (and/or changes thereof), variations in perfusion may be determined. Detecting variations of blood perfusion may allow to increase the accuracy of the vital sign information, e.g., by compensating for the variations of blood perfusion in the algorithm used to determine the vital sign information. Furthermore, determining the variations of blood perfusion may allow to determine precisely systolic blood pulses. Also, vital sign information such as heart rate variability or a sleep state of the subject may be determined, which depend on heart rate or heart beats (including inter-beat-intervals, beat amplitude).

Optionally, the determination of variations of blood variations may be combined with PPG based algorithms. Hence, in a further embodiment, the method comprises determining one or more photoplethysmography, PPG, signals from the one or more obtained detection signals, wherein the one or more determined PPG signals are used in addition to the one or more determined light path characteristics to determine the vital sign information of the subject.

In particular, a PPG signal not taking into account the light path characteristics may be corrected using the one or more LPC. For example, accuracy of the determined PPG signal may be improved by using different calibration curves depending on the determined one or more LPC. For example, depending on the tissue penetration depth ratio of light of different wavelengths (e.g., red light and infrared light), and/or depending on the path length ratio of light of different wavelengths (e.g., red light and infrared light), a calibration curve from a plurality of plurality of calibration curves may be selected. Generally, it is not necessary that the light used for the SpO2 determination has the same wavelength as the light used for the TOF determination. Any information about the tissue and its pulsatile components can be used to improve the SpO2 accuracy. It is preferred to use the same wavelengths though as the information would more directly relate to the improvement in accuracy.

Furthermore, a reliability of the PPG signal or the reliability of a metric derived from the PPG signal may be determined, e.g., by validating if the sensing operates within desired conditions. For example, the one or more TOF signals or the one or more LPC may be used to determine a reliability index of the metric derived from the PPG signal (e.g. a mean absolute error). For example, if the light paths differ strongly, the PPG signal or the metric derived therefrom may be less reliable. Based on the reliability index or a warning, a non-reliable metric may be rejected, which may allow for more accurate clinical decisions.

In another example, accuracy of the PPG signal such as SpO2, may be improved based on a modulating portion of the LPC and/or a ratio of modulating portions of the LPC of light of different wavelengths. For example, a calibration curve for the PPG signal may be selected accordingly. For example, the ratio of modulating path lengths and/or the ratio of modulating penetration depths of light of different wavelengths (e.g., red light and infrared light) may be used to select a calibration curve for the SpO2 value.

Alternatively, the ratio of the modulating LPC may be used as a direct input to a conventional calibration curve, e.g. for SpO2, to retrieve an accurate value of the vital sign (e.g., SpO2).

The one or more TOF signals and the PPG signal may be determined using the same light source and/or the same light detector. Both the one or more TOF signals and the PPG signal may be determined based on the one or more detection signals. Alternatively, different light sources and/or different light detectors may be used to obtain the one or more TOF signals and the PPG signal.

The PPG signal may be determined in direct contact with the subject or remotely, e.g. camera based.

Furthermore, the PPG signal may be a transmissive PPG signal (the light traveling through the tissue) or a reflective PPG signal (the light being reflected by the skin).

In one embodiment, the method further comprises obtaining one or more photoplethysmography, PPG, signals; and improving the accuracy of the one or more obtained photoplethysmography, PPG, signals using the light path characteristics.

Thus, the PPG signal may be determined independently from the TOF signal.

In another embodiment, the method further comprises determining, from the one or more determined light path characteristics, one or more of: differences in anatomy and/or physiology of the subject compared to other subjects; and pressure variations of pressure exerted by the light source and/or light detector. Hereby, it may be of less importance whether the changes in LPC are caused internally (by the hemodynamic status of the patient) or externally (pressure of the probe).

The light path characteristics may vary with the pressure exerted by the light source and/or light detector. For example, the PD may decrease with increasing exerted pressure. Thus, by detecting variations of PD variations in the exerted pressure may be detected. Detecting pressure variations may allow to increase the accuracy of vital sign information, e.g., by compensating for the determined pressure variations in the algorithm used to determine the vital sign information. Furthermore, detecting pressure variations may allow to determine whether the device for determining the vital sign information of the subject is correctly disposed on the subject, i.e., too tightly or too loosely. Furthermore, a user may be guided to dispose correctly the device for determining the vital sign information of the subject on the subject.

The method may additionally comprise the step of obtaining light path characteristics corresponding to the anatomy and/or physiology of other subjects, e.g. for a specific body site, and comparing the obtained light path characteristics of the other subjects with the determined light path characteristics of the subject.

In a further embodiment, the light source comprises two or more light source elements configured to emit light signals at different positions into the tissue of the subject; and/or wherein the light detector comprises two or more light detection elements configured to detect light from different positions of the subject.

The more light sources and/or the more light detectors the system comprises, the more accurate inhomogeneity and other optical properties of the tissue may be captured. Thus, the reliability and/or the accuracy of the vital sign information may be improved. For example, outliers in the vital sign information may be averaged out.

In one embodiment, the two or more light source elements are configured to emit light at different wavelengths; and/or the two or more light detection elements are each configured to detect light at different wavelengths.

By emitting and/or detecting light at different wavelengths, vital sign information may be determined more precisely. Furthermore, additional vital sign information may be determined which cannot be determined from LPC of light at one wavelength only. For example, SpO2 may be determined by emitting and/or detecting red light and infrared light.

BRIEF DESCRIPTION OF THE DRAWINGS

These and other aspects of the invention will be apparent from and elucidated with reference to the embodiments described hereinafter. In the following drawings

Fig. 1 shows a schematic diagram of an embodiment of a system according to the present invention.
Fig. 2 shows a schematic diagram of another embodiment of the system according to the present invention.
Fig. 3 shows a schematic diagram of an embodiment of a method according to the present invention.
Fig. 4 shows a schematic diagram of another embodiment of the method according to the present invention.
Fig. 5 shows a schematic diagram of photons detected over time.
Fig. 6 shows a light path characteristic varying over time.
Fig. 7A and Fig. 7B show different light paths.
Fig. 8A and Fig. 8B shows a schematic diagram of a light path characteristic during diastole and systole.
Fig. 9A and Fig. 9B show a schematic diagram of another light path characteristic for large penetration depth and small penetration depth.
Fig. 10A and Fig. 10B show a schematic diagram of a further light path characteristic for shallow perfusion and deeper perfusion.
Fig. 11A and Fig. 11B show a schematic diagram of yet a further light path characteristic for high perfusion and low perfusion.
Fig. 12 shows a diagram of photon count over time.
Fig. 13 shows diagrams of the photon count over time and distance.
Fig. 14 shows diagrams of different penetration depths and of corresponding photon counts.

DETAILED DESCRIPTION OF EMBODIMENTS

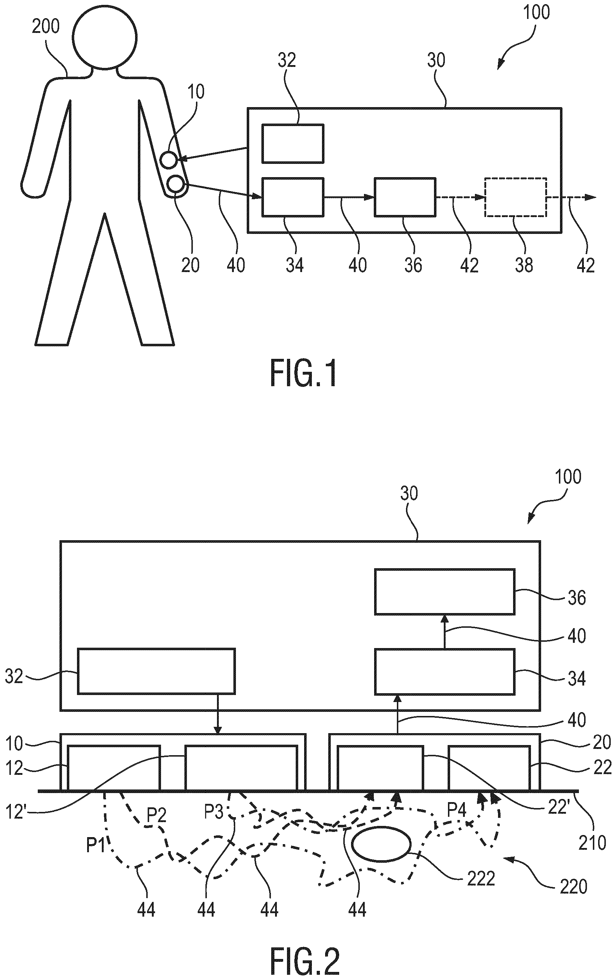

Fig. 1 shows a schematic diagram of an embodiment of a system 100 for determining vital sign information of a subject 200 according to the present invention. The system 100 comprises a light source 10, a light detector 20 and a device 30 for determining vital sign information of the subject 200. The device 30 comprises a control unit 32, a signal input 34 and a processing unit 36.

The light source 10 comprises one or more light source element, e.g. one or more LEDs, which are each configured to emit light at specific wavelengths. For example, the light source 10 comprises a red LED and an infrared LED.

The light source 10 and the light detector 20 are deployed on a body part such as an arm of the subject 200 in direct contact with the skin, e.g. as part of a wearable device. Alternatively, the light source 10 and/or the light detector 20 may be arranged remotely, wherein the distance to the skin may be very small (e.g. a few mm or cm) or larger (e.g. up to a few m).

The light detector 20 is for example a photodiode.

The light source 10 emits a light signal at one or more wavelength into the tissue of the subject 200. The emitted light signal travels along a light path through the tissue of the subject 200, reemerges from the tissue and is detected by the light detector 20. Based on the detected light, the light detector 20 generates a detection signal 40. The device 30 obtains the generated detection signal 40 and determines based on the obtained detection signal 40 vital sign information 42.

The vital sign information 42 provides information on the subject's health state and may be used for monitoring the subject 200. For example, the vital sign information 42 comprises a pulse rate, heart rate, oxygen saturation and/or variation of blood perfusion.

For determining the vital sign information 42, the control unit 32 of the device 30 controls the light source 10 to emit the light signal at the one or more wavelengths. Based on the obtained one or more detection signals 40, the processing unit 34 determines one or more TOF signals. From the one or more TOF signals, the processing unit 34 then determines one or more LPC of the light path along which the light has travelled. Based on the determined LPC, the processing unit 34 then determines vital sign information 42 of the subject 200.

Optionally, the device 30 comprises an output unit 38 which is configured to output the vital sign information 42.

Fig. 2 shows a schematic diagram of another embodiment of the system 200 according to the present invention. The system 200 comprises the light source 10 having two light source elements 12, 12', the light detector 20 having two light detector elements 22, 22' and the device 30 for determining the vital sign information 42 of the subject 200.

The light source elements 12, 12' are placed in direct contact with the skin 210 of the subject 200. The light source elements 12, 12' are controlled by the control unit 32 to emit light into the tissue 220 of the subject 200, wherein each light source element 12, 12' periodically emits a light signal at one wavelength. For example, the light source element 12 emits red light and the light source element 12' emits green light. With each (pulse of) light signal one or more photons P are emitted, which travel along a light path 44 from the respective light source element 12, 12' to one of the light detector elements 22, 22'.

As schematically shown in Fig. 2, the light path 44 depends on the wavelength of the light. In particular, a path length and a tissue penetration depth into the tissue 220 depends on the wavelength of the light. Photons P1 and P2 emitted by the light source element 12 penetrate deeper into the tissue 220 than photons P3 and P4 emitted by the light source element 12'. By penetrating deeper into the tissue 220, the path length of P1 and P2 is larger, too.

Furthermore, the light paths 44 from photons emitted by one light source element 12, 12' differ, too. Hence, light paths 44 of light of the same wavelength differ too. Photon P1 travels from light source element 12 to light detector element 22. P1 travels along a relatively long light path 44, being scattered into various directions. When arriving at reflective bone 222 arranged in the tissue 220 of the subject 200, P1 is forced to pass below the bone 222, which prolongs the path length. In contrast thereto, photon P2 is forced by the bone 222 to travel above the bone 222 through the tissue 220, which results in a shorter path length and a smaller penetration depth than that of P2.

Photons P3 and P4 are emitted by the light source element 12'. As P3 and P4 penetrate less deeply into the tissue 220 than P1 and P2, the corresponding light paths 44 of P3 and P4 are not influenced by the bone 222 (the bone 222 is "not in the way"). The path length and the tissue penetration depth of P3 and P4 is smaller than the path length and the tissue penetration depth of P1 and P2.

Thus, the light paths 44 of the red light and the green light differ. By comparing the TOF information of the red light and the green light, it can be determined that the tissue 220 is inhomogeneous, i.e. in this case, the bone 222 is present.

Optionally, the system 100 additionally determines a PPG signal using the light source 10, the light detector 20 and the device 30. The control unit 32 controls the light source elements 12, 12' to emit light signals which are detected by the light detector elements 22, 22'. Based on the one or more detection signals 40, the processing unit 36 determines a PPG signal by determining the portion of light emerging from the tissue and in particular by determining the portion of light emerging from the tissue relative to the portion of light being absorbed by the tissue 220. Based on the determined PPG signal, the processing unit 36 determines vital sign information 42 (such as SpO2), wherein the accuracy the vital sign information 42 is improved using the LPC.

The device 30 may have a relatively high-power usage compared to conventional PPG devices. The reason is that in conventional PPG devices, the emittance of light is relatively short in order to save power (and thus to maximize battery life in a wearable device). Thus, the light source of conventional PPG devices is off most of the time. The high-power usage of device 30 using TOF may be compensated by using a suitable shape of light signal (e.g., square wave) or activating the TOF functionality (with long pulses) only once in a while, while the PPG signal is determined continuously. This is possible as some of the LPC change only relatively slowly over time.

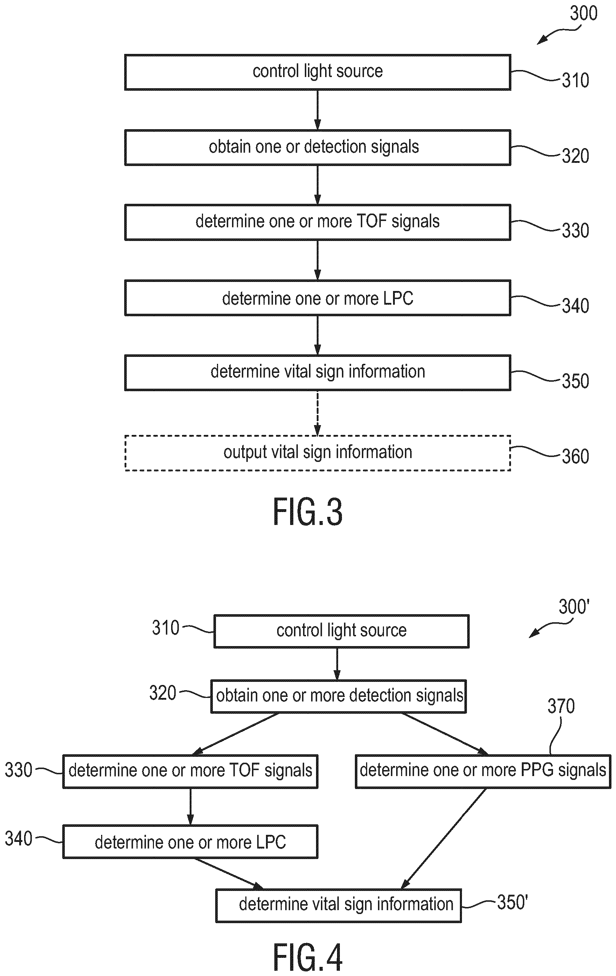

Fig. 3 shows a schematic diagram of an embodiment of method 300 for determining the vital sign information 42 from the subject 200 according to the present invention. The steps of the method 300 may be carried out by the device 30. The method 300 may e.g. be implemented as a computer program running on a computer or processor.

In step 310, the light source 10 is controlled to emit light signals at one or more wavelengths into the tissue 220 of the subject 200. In step 320, one or more detection signals 40 are obtained from the light detector 20. In step 330, one or more TOF signals are determined from the one or more obtained detection signals 40. In step 340, based on the determined one or more TOF signals, one or more LPC of the light path 44 along which the light has travelled are determined. In step 350, vital sign information 42 of the subject 200 is determined based on the one or more determined LPC.

Optionally, method 300 may comprise the additional step 360 of outputting the determined vital sign information 42 of the subject 200.

Fig. 4 shows a schematic diagram of another embodiment of method 300' for determining the vital sign information 42 from the subject 200 according to the present invention. Steps 310, 320 and 340 correspond to steps 310, 320 and 340 of Fig. 3.

Additionally, method 300' comprises the step 370. Step 370 may be carried out before, after or in parallel to step 330 and/or step 340. In step 370, one or more PPG signals are determined based on the one or more detection signals 40 obtained in step 320. In step 350', the vital sign information 42 is determined based on the one or more PPG signals, wherein the accuracy of the one or more PPG signal is improved using the one or more LPC determined in step 340.

Optionally, the method 300' may comprise the step 360, in which the vital sign information 42 is outputted.

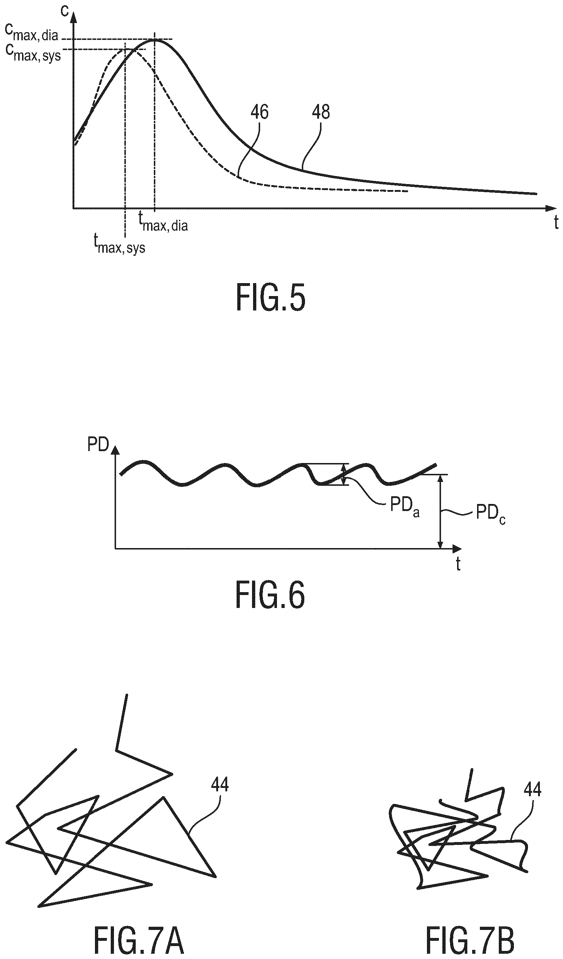

Fig. 5 and 6 schematically show an example for the determination of a LPC using the TOF. For each detected photon P of a light signal, the TOF is determined. Fig. 5 shows a detection signal 40 comprising a number c of detected photons P over a (relative) time t, which corresponds to the TOF of the respective photon P. The number c of detected photons P increases over the time t and decreases after reaching a maximum. The shape and the absolute values of the resulting curve depend on the characteristics of the tissue 220 (e.g., inhomogeneity, bones, etc.), the characteristics of the light signal (e.g., the wavelength) and the point in time the light signal is emitted (relative to working of the cardio-vascular system of the subject 200, e.g., a point in time during systole or diastole).

Fig. 5 shows a curve 46 detected during systole and a curve 48 detected during diastole. The systolic curve 46 is determined based on a light signal emitted during a heartbeat (when blood is pumped) and the diastolic curve 48 is determined based on a light signal between two heart beats. As the blood volume in the blood vessels varies over (absolute) time, the detection signal 40 varies too, resulting in different curves 46, 48.

TOF and subsequently LPC may be determined based on curves 46 and 48. The peak time at which the maximum of photons P are counted gives $t_{max, sys}$ for the curve 46 and $t_{max, dia}$ for the curve 48. The peak time corresponds to the (common) TOF of the light signal. The (common) TOF can be determined over the time, giving a TOF signal.

LPC comprises, e.g., path length information indicating the path length of the light path 44 or tissue penetration depth information indicating the penetration depth PD of light into the tissue 220.

A tissue penetration depth PD may be calculated using $PD = t_{max} * c_{max}$, wherein $c_{max}$ is the maximum number c of counted photons P at $t_{max}$. The resulting PD over time is shown in Fig. 6. As the (common) TOF varies over (absolute) time, PD varies over time too. The path length may be determined by assuming a constant velocity, which corresponds to the speed of light in tissue which can be assumed constant (assuming the tissue is watery and of relatively homogeneous density/humidity).

The unit of PD is generally meter (distance). The depth that is determined is a real depth, with unit meter, but it is mainly "characteristic" in the sense that it characterizes an entire distribution of photons, some of which have traveled farther before being detected, while some of which have traveled shorter distances. Hence, "PD" herein may be understood as "CPD" (characteristic penetration depth).

The PD signal is characterized by an average tissue penetration depth $PD_c$ (signal) and a modulating portion $PD_a$. $PD_c$ corresponds to an average PD over time t. For example, $PD_c$ corresponds to the central line of the oscillating $PD_a$ signal. $PD_a$ corresponds to twice amplitude of the PD signal.

Fig. 7A and 7B schematically show the scattering of a photon along its light path 44. The light path 44 in Fig. 7A shows relatively little scattering and the light path 44 in Fig. 7B shows more scattering. With a higher scattering, the photon changes the direction of flight more often. The more scattering, the light path 44 may be characterized by a longer path length and/or a larger tissue penetration depth. More scattering may be due, e.g., to a higher amount of blood volume in the blood vessels and/or inhomogeneity of the tissue 44.

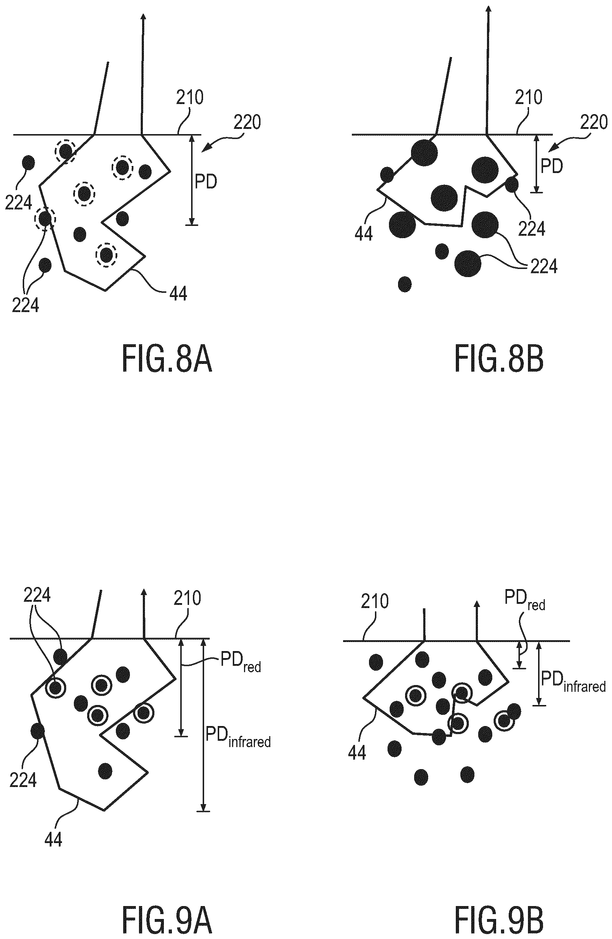

Fig. 8A and Fig. 8B schematically show an exemplary light path 44 of a photon during diastole (Fig. 8A) and systole (Fig. 8B). During diastole, the blood volume in the blood vessels 224 is smaller than during systole. The dashed lines around blood vessels 224 indicate the vessel size during the arrival/presence of the cardio-vascular pressure wave ("systole"), i.e. the systolic diameter, while the spot in the center indicates the vessel size during the diastole, i.e. the diastolic diameter.

As can be seen from Fig. 8A and Fig. 8B, the light path 44 during diastole (Fig. 8A) is longer and the corresponding photon penetrates deeper into the tissue 220 than during systole (Fig. 8B). It shall be noted that photons hitting a blood vessel may pass through (with red and IR there is a very high chance of transmittance due to the low absorption coefficient), but the chance of being absorbed is significantly higher than in the surrounding soft / watery tissue. Figs. 8A and 8B thus illustrate that a larger blood volume in the same tissue volume reduces the characteristic PD due to absorption of light by blood. This is essentially an illustration of the modulated part $PD_a$ of the PD that is illustrated by arrows that indicate the typical (predominant) penetration depth of the photon density distribution (i.e. the peak of the distribution).

Fig. 9A and Fig. 9B schematically show an embodiment in which the accuracy of a PPG signal such as SpO2 may is improved. The PD is determined for light signals at two different wavelengths such as red light and infrared light. The tissue penetration depth of the infrared light $PD_{infrared}$ is larger than the tissue penetration depth of the red light $PD_{red}$, both for shallow perfusion (Fig. 9A) and deeper perfusion (Fig. 9B). For shallow perfusion, both $PD_{red}$ and $PD_{infrared}$ are larger than the respective PD for deeper perfusion (as a result of which the pulsatile vessels may be seen relatively poorly in the infrared channel).

The ratio of $PD_{red}$ to $PD_{infrared}$ may be used to choose a calibration curve which is applied to the SpO2 signal to improve the accuracy thereof. Preferably, the ratio is calculated using the average tissue penetration depth $PD_c$ signal, and therefore the ratio $PD_{c, red} / PD_{c, infrared}$. In another embodiment, the ratio of $PD_{red}$ to $PD_{infrared}$ may be used directly for the calculation of SpO2, e.g. using a look-up table in the form of a formula. The correction may be based on the assumption that PD ratios unequal one result imply SpO2 inaccuracies (if no correction is made). The assumption may be made because in general, pulsatile distributions are similar for different subjects 200. For example, in Fig. 9B, the non-pulsatile layer is barely "seen" by the infrared light.

Figs. 9A and 9B illustrate that even during diastole (both figures show the arterioles with vessels) there may be distinctly different CPD values: due to different concentrations of venous blood, which do not feature systole diastole, which impact the CPD due to absorption of light by the venous blood. This would reduce the $PD_c$ for both wavelengths (differently) as indicated by arrows.

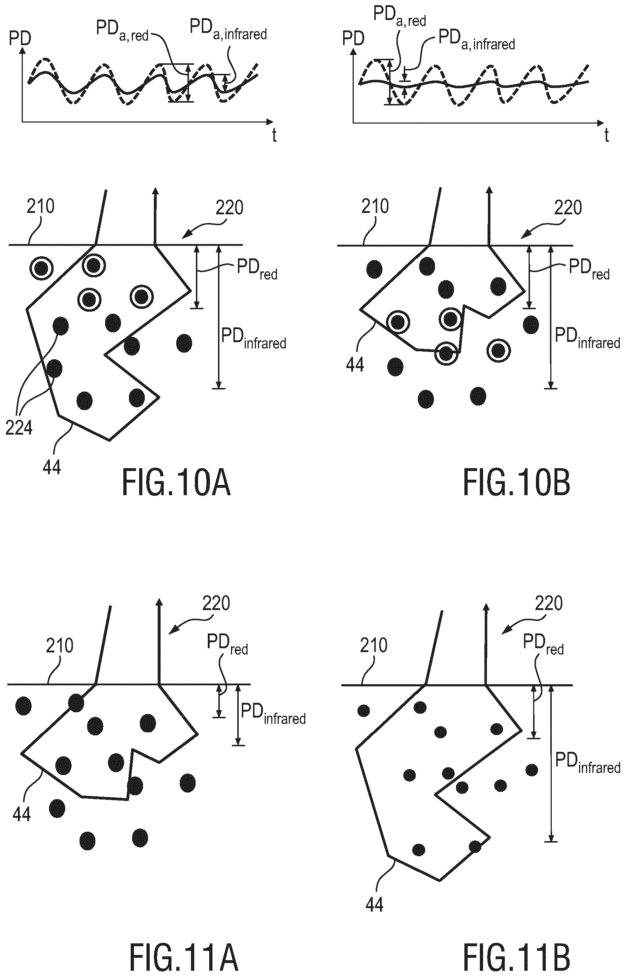

Fig. 10A and Fig. 10B schematically show another embodiment in which the accuracy of a PPG signal such as SpO2 is improved. Similarly to the embodiment shown in Fig. 9A and Fig. 9B, the PD is determined. In contrast to the embodiment shown in Fig. 9A and Fig. 9B, the ratio of the modulating portion $PD_{a, red} / PD_{a, infrared}$ is used to improve the PPG signal.

Figs 10A and 10B illustrate that in a situation where the overall PDs are the same it can be differentiated between the situation with a shallow pulsatile layer (as shown in Fig. 10A) and a deeper pulsatile layer (as shown in Fig. 10B) by observing that the $PD_a$ components are different. This information may then be used to improve the SpO2 accuracy (e.g. via the plurality of calibration curves ...). At this point it is not yet known which are possibly more powerful in terms of improving SpO2 accuracy.

Fig. 9A shows the tissue 220 with shallow perfusion and Fig. 9B shows the tissue 220 with deeper perfusion. The tissue penetration depth PD modulates over the time for both the shallow perfusion and the deeper perfusion, wherein the PD of the infrared light varies less for the deeper perfusion (Fig. 10B). To the contrary, the PD of the red light is similar for the shallow perfusion and the deeper perfusion. Therefore, the ratio $PD_{a, red} / PD_{a, infrared}$ for the shallow perfusion (Fig. 10A) is larger than the ratio in $PD_{a, red} / PD_{a, infrared}$ for the deeper perfusion (Fig. 10B). For example, for the shallow perfusion, exemplary values may be $PD_{a, red} = 2$ and $PD_{a, infrared} = 4$, and for the deeper perfusion, $PD_{a, red} = 0.5$ and $PD_{a, infrared} = 4$, resulting in $PD_{a, red} / PD_{a, infrared} = 0.5$ for the shallow perfusion and $PD_{a, red} / PD_{a, infrared} = 0.125$ for the deeper perfusion. The ratio $PD_{a, red} / PD_{a, infrared}$ is then used to select a calibration curve for to correct the PPG signal.

Alternatively, the term

$$\frac{PDa, red}{PDa, infrared} \quad \frac{PPGred}{PPGinfrared}$$

may be used as input to a conventional PPG calibration curve, with the PPG signal for the red light $PPG_{red}$ and the PPG signal for the infrared light $PPG_{infrared}$.

Fig. 11A and Fig. 11B schematically show an embodiment in which variations in perfusion may be determined. Fig. 11A shows a situation with relative high perfusion and Fig. 11B shows a situation with lower perfusion. Depending on the perfusion, the light is scattered more or less. The higher the perfusion, the more the scattering. By determining LPC, variations in perfusion may be detected. Figs. 11A and 11B thus illustrate that a tissue with more blood results in a smaller PD.

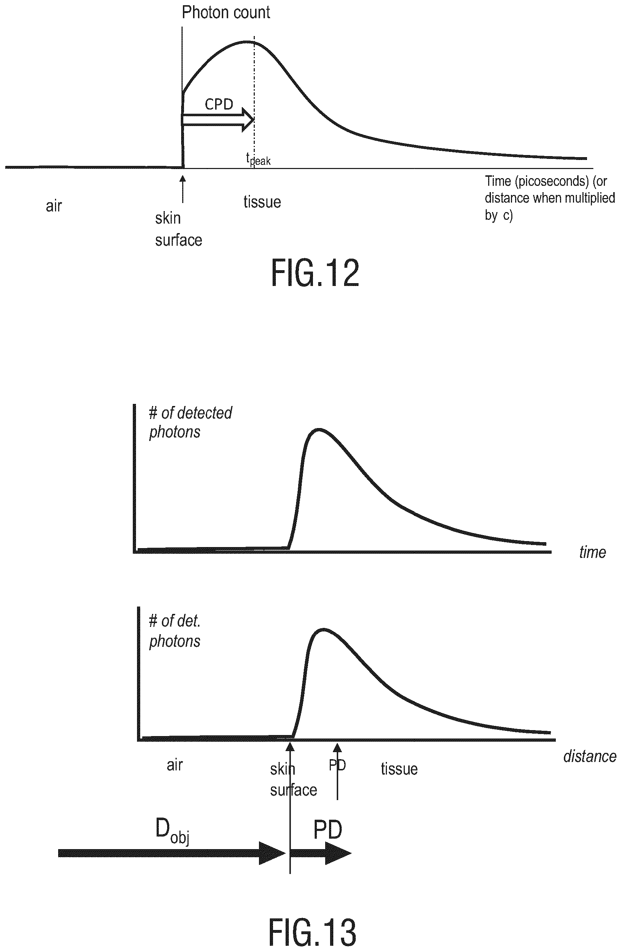

For further explanation of the background of the present invention, Fig. 12 shows a diagram of photon count over time (or distance when time is multiplied by c). The smallest time (distance) at which non-zero photon count is detected can be considered the skin surface. Anything beyond that are photons that have travelled through the tissue and were remitted with a delay. The peak photon count gives $CPD = t_{peak} * c$.

Fig. 13 shows diagrams of photon counts over time (upper diagram) and distance (lower diagram). Typically, in ToF applications, the distance D to the object is of main interest as it is used to generate a 3D image. According to the present invention, the PD and time varying PD is of main interest, which are illustrated in the lower diagram.

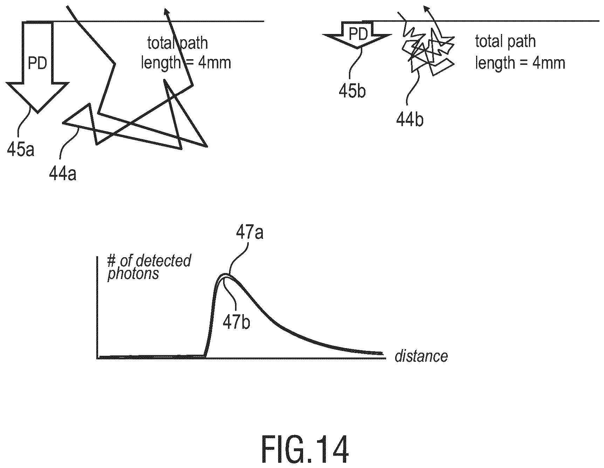

Fig. 14 shows diagram of different PDs 45a, 45b for the same path length despite different light paths 44a, 44b. In the lower diagram corresponding curves 47a, 47b of the number of photons over distance are depicted. It can be derived from this figure that pathlength does not relate to PD as an absolute relationship. The same peak in the ToF photon distribution may still relate to different PDs. Changes in PD due to changes in blood volume fraction (perfusion) may be monitored though, as the scattering properties of the tissue do not change drastically over short time (although they do change with age/-years).

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

For example, it should be noted that the system is not limited to one or two light sources and one or two light detectors. Instead, the system may comprise a plurality of light sources (i.e., two, three, four or more) and/or a plurality of light detectors (i.e., two, three, four or more). It should be furthermore noted that the light sources shown in the embodiments are not limited to a certain wavelength of light but may emit any suitable wavelength.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless communication systems.

Any reference signs in the claims should not be construed as limiting the scope.

**Claims**

1. Method (300) for determining vital sign information (42) of a subject (200), the method (300) comprising:

   - controlling a light source (10) to emit light signals at one or more wavelengths into tissue (220) of the subject (200);
   - obtaining one or more detection signals (40) from a light detector (20) configured to detect light after travelling from the light source (10) through the tissue (220) of the subject (200) to the light detector (20) along a light path (44);
   - determining one or more time of flight, TOF, signals from the one or more obtained detection signals (40);
   - determining, from the one or more TOF signals, one or more light path characteristics of the light path (44) along which the light has traveled; and
   - determining vital sign information (42) of the subject (200) from the one or more determined light path characteristics.

2. Method (300) according to claim 1, wherein determining one or more light path characteristics includes determining one or more of:

   - path length information indicating the path length of the light path (44) along which the light has traveled,
   - path length difference information indicating differences between path lengths of the light paths (44) along which light of different wavelengths has travelled,
   - tissue penetration depth information indicating the penetration depth of light into the tissue (220), and
   - tissue penetration difference information indicating differences between penetration depths into the tissue (220) of light of different wavelengths.

3. Method (300) according to any preceding claim, wherein determining the one or more light path characteristics comprises taking into account one or more of:

   - a peak time, $t_{max}$, at which a maximum of photons of one of the emitted light signals has been detected by the light detector (20); and
   - a maximum number of photons, $c_{max}$, detected at the peak time, $t_{max}$.

4. Method (300) according to any preceding claim, wherein determining vital sign information (42) includes determining or improving the determination of one or more of pulse rate, oxygen saturation, blood perfusion and variations of blood perfusion.

5. Method (300) according to any preceding claim, wherein pulse rate, as vital sign information (42), is determined by detecting periodic variations of a path length from the one or more determined light path characteristics, LPC, or by detecting variations of a penetration depth of light into the tissue (220) from the one or more determined light path characteristics.

6. Method (300) according to any preceding claim, wherein oxygen saturation, as vital sign information (42), is determined or its determination is improved by detecting differences between path lengths of the light paths (44) along which light of different wavelengths has travelled or by detecting differences between penetration depths into the tissue (220) of light of different wavelengths.

7. Method (300) according to any preceding claim, wherein variations of blood perfusion, as vital sign information (42), is determined by detecting the path length of the light paths (44) along which light has travelled or by detecting the penetration depth of light into the tissue (220).

8. Method (300) according to any preceding claim,

   further comprising determining one or more photoplethysmography, PPG, signals from the one or more obtained detection signals (40), wherein the one or more determined PPG signals are used in addition to the one or more determined light path characteristics to determine the vital sign information (42) of the subject (200).

9. Method (300) according to any preceding claim, further comprising

   - obtaining one or more photoplethysmography, PPG, signals; and
   - improving the accuracy of the one or more obtained PPG signals using the light path characteristics.

10. Method (300) according to any preceding claim, further comprising determining, from the one or more determined light path characteristics, LPC, one or more of:

    - differences in anatomy and/or physiology of the subject (200) compared to other subjects (200); and
    - pressure variations of pressure exerted by the light source (10) and/or light detector (20).

11. Device (30) for determining vital sign information (42) of a subject (200), the device (30) comprising:

    - a control unit (32) configured to control a light source (10) to emit light signals at one or more wavelengths into tissue (220) of the subject (200);
    - a signal input unit (34) configured to obtain one or more detection signals (40) from a light detector (20) configured to detect light after travelling from the light source (10) through the tissue (220) of the subject (200) to the light detector (20) along a light path (44); and
    - a processing unit (36) configured to

      determine one or more time of flight, TOF, signals from the one or more obtained detection signals (40);
      determine, from the one or more TOF signals, one or more light path characteristics of the light path (44) along which the light has traveled; and
      determine vital sign information (42) of the subject (200) from the one or more determined light path characteristics.

12. System (100) for determining vital sign information (42) of a subject (200), the system (100) comprising:

    - a light source (10) configured to emit light signals at one or more wavelengths into tissue (220) of the subject (200);
    - a light detector (20) configured to detect light after travelling from the light source (10) through the tissue (220) of the subject (200) to the light detector (20) along a light path (44) and generate one or more detection signals (40) based on the detected light; and
    - a device (30) according to claim 11 configured to determine vital sign information (42) of the subject (200) from the one or more detection signals (40).

13. System (100) according to claim 12,

    wherein the light source (10) comprises two or more light source elements (12, 12') configured to emit light signals at different positions into the tissue (220) of the subject (200); and/or
    wherein the light detector (20) comprises two or more light detection (22, 22') elements configured to detect light from different positions of the subject (200).

14. System (100) according to claim 13,

    wherein the two or more light source elements (12, 12') are configured to emit light at different wavelengths; and/or
    wherein the two or more light detection elements (22, 22') are each configured to detect light at different wavelengths.

15. Computer program comprising program code means for causing a computer to carry out the steps of the method (300) as claimed in any one of claims 1 to 10 when said computer program is carried out on the computer.

200

100

32

30

10

20  40  34  40  36  42  38  42

# FIG.1

100

30

36

40

34

40

32

10
12
12'

20
22
22'

P2    P3    P4    210

P1    44    44    44    44    222    220

# FIG.2

FIG.3

FIG.4

FIG.5

FIG.6

FIG.7A

FIG.7B

210

220

PD

224

224

44

## FIG.8A

210

220

PD

44

224

224

## FIG.8B

224

210

PD_red

PD_infrared

224

44

## FIG.9A

210

PD_red

PD_infrared

44

## FIG.9B

FIG.10A

FIG.10B

FIG.11A

FIG.11B

FIG.12

FIG.13

total path
length = 4mm

PD

45a

44a

total path
length = 4mm

PD

45b

44b

47a

# of detected
photons

47b

distance

# FIG.14

Europäisches
Patentamt

European
Patent Office

Office européen
des brevets

# EUROPEAN SEARCH REPORT

Application Number

EP 24 19 8722

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2018/168492 A1 (VERMEULEN OLAF THOMAS JOHAN ANTONIE [NL]) 21 June 2018 (2018-06-21) * paragraph [0005] - paragraph [0092] * ----- | 1-15 | INV. A61B5/024 A61B5/026 A61B5/1455 |
| Y | DELPY D T ET AL: "ESTIMATION OF OPTICAL PATHLENGTH THROUGH TISSUE FROM DIRECT TIME OF FLIGHT MEASUREMENT", PHYSICS IN MEDICINE AND BIOLOGY, INSTITUTE OF PHYSICS PUBLISHING, BRISTOL GB, vol. 33, no. 12, 1 January 1988 (1988-01-01), pages 1433-1442, XP000195639, ISSN: 0031-9155, DOI: 10.1088/0031-9155/33/12/008 * the whole document * * figures 1-5 * ----- | 1-15 | |
| A | US 5 987 351 A (CHANCE BRITTON [US]) 16 November 1999 (1999-11-16) * column 1, line 10 - column 11, line 32 * * figures 1-11 * ----- | 1-15 | |
| A | US 6 453 183 B1 (WALKER STEPHEN D [US]) 17 September 2002 (2002-09-17) * the whole document * ----- | 1-15 | |

TECHNICAL FIELDS SEARCHED (IPC)

A61B

The present search report has been drawn up for all claims

| Place of search | Date of completion of the search | Examiner |
|---|---|---|
| Berlin | 26 January 2025 | Abraham, Volkhard |

EPO FORM 1503 03.82 (P04C01)

## ANNEX TO THE EUROPEAN SEARCH REPORT
## ON EUROPEAN PATENT APPLICATION NO.

EP 24 19 8722

This annex lists the patent family members relating to the patent documents cited in the above-mentioned European search report.
The members are as contained in the European Patent Office EDP file on
The European Patent Office is in no way liable for these particulars which are merely given for the purpose of information.

26-01-2025

| Patent document cited in search report | | | Publication date | Patent family member(s) | | Publication date |
|---|---|---|---|---|---|---|
| US 2018168492 | A1 | | 21-06-2018 | BR 112017025708 | A2 | 07-08-2018 |
| | | | | CN 107666860 | A | 06-02-2018 |
| | | | | EP 3302253 | A1 | 11-04-2018 |
| | | | | JP 6878312 | B2 | 26-05-2021 |
| | | | | JP 2018519889 | A | 26-07-2018 |
| | | | | US 2018168492 | A1 | 21-06-2018 |
| | | | | WO 2016193048 | A1 | 08-12-2016 |
| US 5987351 | A | | 16-11-1999 | CA 2209240 | A1 | 11-07-1996 |
| | | | | CN 1169665 | A | 07-01-1998 |
| | | | | DE 69627477 | T2 | 18-03-2004 |
| | | | | EP 0808124 | A1 | 26-11-1997 |
| | | | | EP 0906052 | A1 | 07-04-1999 |
| | | | | JP 3725156 | B2 | 07-12-2005 |
| | | | | JP H10511875 | A | 17-11-1998 |
| | | | | US 5987351 | A | 16-11-1999 |
| | | | | US 6526309 | B1 | 25-02-2003 |
| | | | | US 2004054290 | A1 | 18-03-2004 |
| | | | | US 2006241502 | A1 | 26-10-2006 |
| | | | | US 2009030327 | A1 | 29-01-2009 |
| | | | | WO 9620638 | A1 | 11-07-1996 |
| | | | | WO 9720494 | A1 | 12-06-1997 |
| US 6453183 | B1 | | 17-09-2002 | US 6453183 | B1 | 17-09-2002 |
| | | | | WO 2004086966 | A1 | 14-10-2004 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016166651 A1